# EUROPEAN PATENT SPECIFICATION

(11) **EP 0 487 557 B1**
(45) Date of publication and mention of the grant of the patent: **14.02.1996**
(21) Application number: 90911518.0
(22) Date of filing: 13.08.1990
(51) Int. Cl.: D21C 9/10, C12S 3/08

(54) **IMPROVEMENT OF PULP BLEACHING**
VERBESSERUNG BEIM BLEICHEN VON PULPE
AMELIORATION DU BLANCHIMENT D'UNE PATE

(30) Priority: 14.08.1989 US 393716
(43) Date of publication of application: 03.06.1992
(73) Proprietor: GENENCOR INTERNATIONAL EUROPE OY, SF-00241 Helsinki (FI)
(72) Inventor: KRUUS, Ilkka, SF-02320 Espoo (FI); LAINE, Jaakko, SF-02460 Kantvik (FI); KOLJONEN, Marja, SF-00360 Helsinki (FI)
(74) Representative: Armitage, Ian Michael
(86) International application number: FI9000194
(87) International publication number: WO9102840

(56) References cited:
- EP-A- 0 386 888
- Biotechnology and Bioengineering, Vol. 32, July 1988 M.G. PAICE et al: "Viscosity-Enhancing Bleaching of Hardwood Kraft Pulp with Xylanase from a Cloned Gene", pp. 235 - 239
- VIIKARI L. et al, "Fourth International Symposium on Wood and Pulping Chemistry", Paris (1987), p. 151-154
- CHAUVET J.-M. et al, "Fourth International Symposium on Wood and Pulping Chemistry", Paris (1987), pp.325-327
- R.P. SINGH "The Bleaching Of Pulp", 1979, Tappi Press, Atlanta, p. 159, p. 171
- Dissertation Abstracts International, Vol. 48, No. 7, January 1988, Abstract No. DA8721908, MEAGHER, MICHAEL MARTIN, "Characterization, kinetics, and subsite mapping of Aspergillus niger glucoamylases I and II, and partial purification and characterization of a Chainia endo-xylanase".

## Description

### Field of the invention

This invention relates to a method for the bleaching of oxygen delignified wood derived kraft pulp, including an enzyme treatment. Specifically, this invention relates to the use of hemicellulose hydrolysing enzyme with little or no cellulase activity.

### Background of the invention

Oxygen bleaching of pulp in its different forms is known and it has been described e.g., in the publication The Bleaching of Pulp, by Singh R., P., Tappi Press 1979,pages 159-209.

The increasing price of energy and the demand for protection of the environment have made it desirable to substitute chemical bleaching processes of pulp with processes demanding less energy, which would, in addition, make it possible to burn all or at least the main part of the waste liquor from bleaching plants in the conventional burning process of alkaline cooking. Oxygen delignification of pulp by using sodium hydroxide as an effective alkali directly after cooking is used today in many paper mills. This process makes it possible to diminish the amount of chlorine containing agents and sodium hydroxide used in bleaching plants and to recycle and burn about half of the dry substances of the bleaching effluents. Extended oxygen delignification, however, causes extensive depolymerization of carbohydrates, thus diminishing the paper properties of pulp. It has not, as yet, been determined how to extend the delignification of pulp by using diminished amounts of chlorine, sodium hydroxide and oxygen and make it possible to burn as much as possible of the spent bleaching liquors.

The treatment of unbleached kraft pulps with hemicellulases is described by Viikari L. et al, 4th International Symposium on Wood and Pulping Chemistry, Paris 1987, pages 151-154; and in Biotechnology and Bioengineering, Vol. 32, 1988, pages 235-239, Paice M.G. et al.

### Summary of the invention

The object of this invention is to improve the bleaching of oxygen delignified wood derived kraft pulp by means of enzyme treatment. The invention is characterized by adding to the bleaching process a hemicellulose hydrolyzing enzyme or enzyme preparation as described in claim 1.

Preferably, the hemicellulase enzyme or enzyme preparation is substantially free from cellulase activity.

According to the invention, hemicellulase enzymes are used in conjunction with oxygen treatment to reduce the amount of chlorine-containing agents and sodium hydroxide used in pulp bleaching processes. The enzyme treatment is performed after the oxygen stage.

### Description of the invention

This invention is based on the discovery that pure hemicellulose-hydrolyzing enzymes or enzyme preparations containing hemicellulose-hydrolyzing enzymes can be used in conjunction with traditional bleaching of oxygen delignified kraft pulp to decrease the amount of chlorine-containing and alkaline agents used during the process.

According to the invention, enzyme preparations, most preferably containing hemicellulase, are used in conjunction with the standard bleaching procedure. It is preferred that the hemicellulase enzyme or preparation used is substantially free from cellulases. As used herein, the term cellulases refers to enzymes which are able to dissolve crystalline cellulose and to liberate therefrom sugars or oligosaccharides. The hemicellulase derived from Chainia sp. NCL 82-5-1 (ATCC No. 53812) is such an enzyme. The hemicellulase produced by that strain, which is the subject of EP-A-0 353 342, has a low molecular weight active xylanase unit capable of good penetration into wood fibers where it can selectively degrade xylan. The enzyme according to the invention can also be obtained in such a way that a cellulase/hemicellulase preparation produced by Trichoderma longibrachiatum is purified by removing all or the main part of the cellulase activity.

The suitable enzyme dosage calculated as xylanase is about 0.1-100 Units (U) per gram of dry solids (d.s.) of the pulp. The preferred level of enzyme is 0.5-25 U/g d.s. of pulp.

The xylanase activity of the enzyme preparation is determined as follows:

To 1 ml of xylan solution (1%, Sigma No.: X-0376, prepared in 50 °C mM Na citrate buffer, pH 5.3), 1 ml of an enzyme suitably diluted in the same buffer is added. The solution is incubated at 50 °C in a water bath for 30 minutes. The reaction is stopped by adding 3 ml of DNS reagent (3,5-dinit-rosalicylate reagent) and the color was developed by cooking the sample for 5 minutes. The absorbance was measured at a wave length of 540 nm. One enzyme unit liberates one micromole of reducing sugar calculated as xylose per one minute under the assay conditions.

The cellulase activity of enzyme preparations was determined as filter paper activity (Ghose, T., K. et al., Symposium of Enzymatic Hydrolysis of Cellulose, Bailey M., Enari, T.M., Linko, M., Eds. (SITRA, Aulanko, Finland, 1975, p. 111 to 136) as follows:

A piece of filter paper (Whatman 1, 50 mg) was added to 1 ml of acetate buffer (0.05 M NaAc, pH 4.8). 1 ml of a suitably diluted enzyme solution was added. The solution was incubated for one hour at 50 °C. The reaction was stopped by adding 3 ml of DNS reagent, and the color was developed and measured as in the xylanase determination. One activity unit liberates 1 micromole of reducing sugars calculated as glucose per one minute.

The hemicellulase enzyme or enzyme preparation is added after treatment with oxygen.

The treatment can be carried out within the pH ranges from about 2 to about 10, preferably from about 4 to about 8, depending e.g. on the origin and the properties of the hemicellulase enzyme. Treatment time depends on the enzyme dosage and the treatment conditions, and it can range from 10 minutes to one day, preferably from 30 minutes to 8 hours. The temperature during the enzyme treatment may vary from about 10 to about 90 °C, preferably from about 25 to 70°C.

The method according to the invention will be illustrated by means of the following examples. The meaning of the examples is not to restrict the invention but to show the working of some embodiments according to this invention.

### Examples

Enzyme of the example 1 was prepared by Chainia sp. NCL 82-5-1 strain. This strain was deposited with the ATCC as No. 53812 on July 27, 1988. Spores of Chainia sp. NCL 82-5-1 strain preserved on PDA slants face was transferred to sterile medium (e.g. 5% wheat bran, 1% yeast extract, pH 7.0).

The medium was cultured in shake flasks for about 3 to 5 days at 30 °C in vigorous shaking speed (50-100 ml liquid, in 250 ml flask, 200 rpm). The liquid was filtered or centrifuged clear. About 10 U/ml of xylanase was produced when wheat bran was used, more xylanase (at least 25 U/ml) was produced when pure xylan base was used. The enzyme thus produced was substantially free from cellulase.

The enzyme used in Example 2 was Multifect K (Cultor Ltd.) produced by the fungus Trichoderma longibrachiatum. The xylanase activity in the preparation was 5,200 U/ml and cellulase activity 4 U/ml.

### Example 1

Oxygen Bleaching I of Birch Pulp Finnish birch sulfate pulp was bleached; the original kappa number was 20.5. A bleaching sequence 0₂-enzyme-D/C-E-D-E-D was used. The amount of chlorine dioxide was 90% and the amount of chlorine 10% calculated as active chlorine in the D/C stage.

The oxygen stage was performed by using the following conditions:
- temperature: 90 °C
- time: 30 min.
- 0₂: 3 bar
- consistency: 10%
- NaOH: 2%

After the oxygen stage, the pulp was divided into two parts. The control pulp was normally bleached, but in order to make the conditions of the treatment as comparable to the enzyme treatment as possible, the control pulp was held at 55 °C and at pH 6 for four hours.

The enzyme treatment of the pulp was performed after oxygen bleaching but before the D/C stage by using the following conditions:
- temperature: 55 °C
- time: 4 hours
- pH: 6
- consistency: 10%
- enzyme dosage as xylanase: 25 U/g of pulp d.s.

After enzyme treatment (or after reference treatment of the control pulp) the pulp was washed and-the final bleaching using the sequence mentioned above was performed. At the D/C stage an active chlorine dosage of 0.2 x kappa % d.s. of pulp was used.

In the four last stages the dosages of chemicals were as follows: El, 0.1 x kappa % d.s. of pulp; D1, 2.5 % d.s. of pulp; E2, 0.8 % d.s. of pulp; D2, 1.0 % of d.s. of pulp.

The results of the experiments are shown in Table 2.

**Table 1**

| Bleaching Experiments/Oxygen Bleaching I/Birch Pulp | | |
|---|---|---|
| | Control | Enzyme Treated |
| Kappa number after oxygen stage | 14.6 | 14.6 |
| Kappa number after enzyme treatment | 13.6 | 11.7 |
| Brightness % | 89.6 | 92.1 |
| Viscosity kg/dm³ | 1015 | 1045 |
| TCl mg/kg | 291 | 252 |
| Yield enzyme treatment + bleaching | 91.8 | 90.9 |

The time of enzyme treatment was moderate (4 hours), yet the brightness increased 2.5 % units, even though the original brightness was almost 90%. Additionally, the yield loss was insignificant.

### Example 2

Oxygen Bleaching II of Birch Pulp Finnish birch sulfate pulp (original kappa number 20.8) was bleached. The bleaching sequence used was 0₂ enzyme-D/C-E-D-E-D and for the reference pulp 02-D/C-E-D-E-D. The ratio of chlorine dioxide to chlorine in the D/C stage was 90:10 calculated on the basis of active chlorine.

The oxygen stage was performed as described in Example 1.

After the oxygen stage the pulp was divided into two parts. One part of the oxygen treated pulp was treated before the D/C stage with enzyme by using the following reaction conditions:
- temperature: 45 °C
- time: 4 hours
- pH: 5
- consistency: 10%
- enzyme dosage as xylanase: 5 U/g pulp d.s.

The enzyme used in the treatment was Multifect K produced by the fungus Trichoderma longibrachiatum.

The other part of the oxygen treated pulp (the control pulp) was treated before the D/C stage using the same conditions as were used in the enzyme treatment but no enzyme was added to the mixture.

After the enzyme treatment (or after the reference treatment of the control pulp) the pulp was washed and the final bleaching was performed as described in Example 1.

The results of the experiments are shown in Table 2.

**Table 2**

| Bleaching Experiments/Oxygen Bleaching V/Birch Pulp | | |
|---|---|---|
| | Control | Treated |
| Kappa number after oxygen stage | 14.8 | 14.8 |
| Kappa number after enzyme treatment | 13.7 | 12.0 |
| Brightness % | 89.3 | 91.3 |
| Viscosity kg/dm3 | 1015 | 1020 |
| TCl mg/kg | 280 | 270 |
| Yield enzyme treatment + bleaching | 91.7 | 91.1 |

Despite the fact that the time of the enzyme treatment was moderate (4 hours) the brightness of the pulp increased 2.0%, even though the original brightness was almost 90%. Additionally, the yield loss was insignificant.

### Example 3

Bleaching of Pine Kraft Pulp

Finnish oxygen delignified pine kraft pulp (original kappa 17,2) was bleached after enzyme treatment and for reference without enzyme treatment (control) with the bleaching sequence D-E1-D1-E2-D2. Other conditions as mentioned in the example 1.

The enzyme preparations used in the experiment were Multifect K (Cultor Ltd.) produced by the fungus Trichoderma longibrachiatum.

The reaction conditions used in the enzyme treatment before conventional bleaching were as follows:
- temperature 45 °C
- time 2 hours
- pH 5,7
- consistency 10%
- enzyme dosage 5 xylanase U/g pulp d.s.

The control pulp was treated before bleaching using the same conditions as in the enzyme treatment but no enzyme was added to the mixture.

The results of experiments are shown in table 3.

**Table 3**

| Bleaching Experiments/Oxygen delignified pine pulp | | |
|---|---|---|
| | Control | Trichoderma |
| Enzyme dosage (xylanase U/g pulp) | 0 | 5 |
| Brightness (%) | 87.6 | 88.7 |

The enzyme treatment improves the bleachability of oxygen delignified kraft pulp even when only chlorine dioxide is used in the first bleaching stage. Oxygen delignified pulp is difficult to bleach to high brightness levels with high chlorine dioxide amounts in the first necessary stage. High amounts of chlorine dioxide in the first stage are needed if the generation of chlorinated organic compounds is to be decreased during bleaching.

A conclusion can be drawn especially from example 1 that by using the enzyme according to this invention in connection with oxygen bleaching, it may be possible to omit the stages E2 and D2. This would result in remarkable savings in investments when building a new bleaching plant.

From the examples it can also be concluded that a target brightness of 85-90% can be reached without the first D/C stage in the conventional bleaching if the pulp is treated with enzymes after the oxygen treatment. If so, the final bleaching after the oxygen stage and enzyme treatment would be the following: E-D-E-D. This means that more of the material dissolved in the bleaching process could be burned because there would be no drawback to burning the material dissolved in the E1 stage.

## Claims

1. A method for the bleaching of oxygen delignified wood derived kraft pulp, characterised in that said method includes a step in which the pulp is treated with a hemicellulose hydrolysing enzyme or enzyme preparation, said enzyme being derived from the genus Trichoderma (or from Chainia sp ATCC 53812.

2. A method according to claim 1 wherein said hemicellulose-hydrolyzing enzyme or enzyme preparation is added prior to the bleaching stage.

3. A method according to claim 1, wherein said hemicellulose-hydrolyzing enzyme contains less than 0.5% (i.e. 1/200) of cellulase activity.

4. A method according to any one of the preceding claims wherein said hemicellulose-hydrolyzing enzyme is produced by Chainia sp. ATCC 53812.

5. A method according to claim 4 wherein said hemicellulose-hydrolyzing enzyme is substantially free from cellulose-hydrolyzing enzyme.

6. A method according to claim 4 wherein said hemicellulose-hydrolyzing enzyme is added in an amount of about 0.1-100 U/g calculated on the dry solids of the pulp.

7. The method according to claim 6 wherein the hemicellulose-hydrolyzing enzyme is added in an amount of about 0.5-25 U/g calculated on the dry solids of the pulp.

8. A method according to claim 4 wherein the hemicellulose-hydrolyzing enzyme treatment is carried out within the pH range from about 2 to about 10; at about 10 to 90°C and the time of enzyme treatment is about 10 min. to 24 h.

9. The method according to claim 8 wherein the hemicellulose-hydrolyzing enzyme treatment is carried out at a temperature of from about 25 to about 70°C for a time period of from about 0.5 to about 8 hours and at a pH range from about 4 to about 8.

10. A method according to any one of the preceding claims wherein the pulp consistency during treatment with hemicellulose hydrolysing enzyme or enzyme preparation is 10%.

## Patentansprüche

1. Verfahren zum Bleichen von mit Sauerstoff delignifiziertem, von Holz stammendem Kraftzellstoff, dadurch gekennzeichnet, daß das Verfahren einen Schritt umfaßt, bei dem der Zellstoff mit einem Hemizellulose hydrolysierenden Enzym oder Enzympräparat behandelt wird, wobei das Enzym von der Gattung Trichoderma oder von Chainia sp. ATCC 53812 stammt.

2. Verfahren nach Anspruch 1, worin das Hemizellulose hydrolysierende Enzym oder Enzympräparat vor dem Bleichschritt zugegeben wird.

3. Verfahren nach Anspruch 1, worin das Hemizellulose hydrolysierende Enzym weniger als 0,5% (d.h. 1/200) Zellulaseaktivität aufweist.

4. Verfahren nach einem der vorangehenden Ansprüche, worin das Hemizellulose hydrolysierende Enzym von Chainia sp. ATCC 53812 erzeugt wird.

5. Verfahren nach Anspruch 4, worin das Hemizellulose hydrolysierende Enzym im wesentlichen frei von Zellulose hydrolysierendem Enzym ist.

6. Verfahren nach Anspruch 4, worin das Hemizellulose hydrolysierende Enzym in einer Menge von etwa 0,1-100 U/g, berechnet auf Basis der trockenen Feststoffe im Zellstoff, zugegeben wird.

7. Verfahren nach Anspruch 6, worin das Hemizellulose hydrolysierende Enzym in einer Menge von etwa 0,5-25 U/g, berechnet auf Basis der trockenen Feststoffe im Zellstoff, zugegeben wird.

8. Verfahren nach Anspruch 4, worin die Behandlung mit Hemizellulose hydrolysierendem Enzym im pH-Bereich von etwa 2 bis etwa 10 bei etwa 10 bis 90°C durchgeführt wird und die Dauer der Enzymbehandlung etwa 10 min bis 24 h beträgt.

9. Verfahren nach Anspruch 8, worin die Behandlung mit Hemizellulose hydrolysierendem Enzym bei einer Temperatur von etwa 25 bis etwa 70°C, über einen Zeitraum von etwa 0,5 bis etwa 8 Stunden und in einem pH-Bereich von etwa 4 bis etwa 8 durchgeführt wird.

10. Verfahren nach einem der vorhergehenden Ansprüche, worin die Zellstoffkonsistenz während der Behandlung mit Hemizellulose hydrolysierendem Enzym oder Enzympräparat 10% beträgt.

## Revendications

1. Méthode de blanchiment de pulpe de kraft dérivée de bois délignifié à l'oxygène, caractérisée en ce que ladite méthode inclut une étape dans laquelle la pulpe est traitée avec une enzyme hydraulisant l'hémicellulose ou une préparation d'enzyme, et ladite enzyme étant dérivée du gène Trichoderma ou de Chainia sp ATCC 53812.

2. Méthode selon la revendication 1, dans laquelle ladite enzyme hydraulisant l'hémicellulose ou ladite préparation d'enzyme est ajoutée avant l'étape de blanchiment.

3. Méthode selon la revendication 1, dans laquelle ladite enzyme hydraulisant l'hémicellulose contient moins de 0,5 % (c'est-à-dire 1/200) d'activité cellulase.

4. Méthode selon l'une quelconque des revendications précédentes dans laquelle ladite enzyme hydraulisant l'hémicellulose est produite par Chainia sp. ATCC 53812.

5. Méthode selon la revendication 4, dans laquelle ladite enzyme hydraulisant l'hémicellulose est substantiellement exempte d'enzyme hydraulisant la cellulose.

6. Méthode selon la revendication 4, dans laquelle ladite enzyme hydraulisant l'hémicellulose est ajoutée à une quantité d'environ 0,1 à 100 U/g calculée sur les solides secs de la pulpe.

7. Méthode selon la revendication 6, dans laquelle l'enzyme hydraulisant l'hémicellulose est ajoutée en une quantité d'environ 0,5 à 25 U/g calculé sur les solides secs de la pulpe.

8. Méthode selon la revendication 4, dans laquelle le traitement est mis en oeuvre dans l'intervalle de pH d'environ 2 à environ 10; à environ 10 à 90°C. et le temps de traitement à l'enzyme est d'environ 10 mn à 24 h.

9. Méthode selon la revendication 8, dans laquelle le traitement à l'enzyme hydraulisant l'hémicellulose est mis en oeuvre à une température d'environ 25 à environ 70° C pendant une période de temps d'environ 0,5 à environ 8 heures et à un pH dans l'intervalle d'environ 4 à environ 8.

10. Méthode selon l'une quelconque des revendications précédentes dans laquelle la consistance de la pulpe pendant le traitement avec enzyme hydraulisant l'hémicellulose ou la préparation d'enzyme est de 10 %.
